Europäisches Patentamt

(19) European Patent Office

Office européen des brevets

(11) EP 0 556 244 B1

(12) **FASCICULE DE BREVET EUROPEEN**

(45) Date de publication et mention
de la délivrance du brevet:
**13.03.1996 Bulletin 1996/11**

(21) Numéro de dépôt: **91919520.6**

(22) Date de dépôt: **28.10.1991**

(51) Int. Cl.⁶: **A61L 2/26**, A23L 3/10, A23L 3/14

(86) Numéro de dépôt international: **PCT/FR91/00848**

(87) Numéro de publication internationale:
**WO 92/08498 (29.05.1992 Gazette 1992/12)**

(54) **DISPOSITIF DE MAINTIEN D'OBJETS A L'INTERIEUR D'UN TAMBOUR TOURNANT**

VORRICHTUNG ZUM HALTEN VON GEGENSTÄNDEN INNERHALB EINES DREHTROMMELS

HOLDER FOR OBJECTS INSIDE A ROTATING DRUM

(84) Etats contractants désignés:
**AT BE CH DE DK ES FR GB IT LI NL SE**

(30) Priorité: **08.11.1990 FR 9013883**

(43) Date de publication de la demande:
**25.08.1993 Bulletin 1993/34**

(73) Titulaire: **BARRIQUAND STERIFLOW
F-42300 Roanne (FR)**

(72) Inventeurs:
• **ROUMAGNAC, Jean-Patrick
F-42120 Le Coteau (FR)**
• **NAVEROS, Francisco
F-42300 Roanne (FR)**

(74) Mandataire: **Dronne, Guy et al
Cabinet Beau de Loménie
158, rue de l'Université
F-75340 Paris Cédex 07 (FR)**

(56) Documents cités:
**AU-A- 27 472          FR-A- 2 481 888**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**Description**

La présente invention a pour objet un dispositif de maintien d'objets à l'intérieur d'un tambour tournant.

De façon plus précise, l'invention concerne un dispositif pour maintenir des objets qui sont placés à l'intérieur du tambour tournant d'appareils tels que des stérilisateurs ou analogues.

La stérilisation d'objets ou de produits de l'industrie alimentaire ou pharmaceutique, tels que des boîtes, bocaux barquettes, flacons, etc..., est souvent réalisée dans des autoclaves dits discontinus dans lesquels on introduit les produits à traiter puis on les en extrait après l'achèvement du traitement. Ces produits à traiter sont disposés dans des paniers, des plateaux empilables ou éléments similaires. Pour certains de ces produits, le traitement thermique de stérilisation doit être effectué avec un mouvement rotatif du produit à l'intérieur de son emballage. Ce mouvement de rotation est produit par la rotation, à l'intérieur de l'autoclave, d'un tambour enfermant des paniers remplis des produits à stériliser. Le même type de problème se pose pour égoutter ou basculer certains produits sur un appareil autre qu'un autoclave qui est alors appelé égoutteur ou basculeur. Cette opération permet l'élimination de l'eau déposée sur ces produits par gravité lors de la stérilisation.

Le brevet français 86 02046 décrit en détails un exemple d'un tel autoclave de stérilisation à tambour tournant.

Lors de la rotation du tambour il est nécessaire d'éviter tout mouvement des produits, ce qui pourrait entraîner leur endommagement ou leur casse, en les immobilisant à l'intérieur du tambour à l'aide d'un dispositif de maintien. En général, les produits individuels sont empilés dans des bacs ou placés sur des plateaux et c'est l'ensemble de ces bacs ou plateaux qu'il y a lieu de maintenir à l'intérieur du tambour. Pour maintenir en place les objets à l'intérieur d'un tambour tournant, on a déjà proposé, comme dans la demande de brevet français FR-A-2 605 226, de commander des plateaux de maintien des objets à l'aide de vérins agissant perpendiculairement au plateau de maintien. Une telle solution permet certes d'obtenir le maintien des objets à l'intérieur du tambour, sans intervention manuelle, mais elle n'est pas satisfaisante dans la mesure où le maintien effectif des objets dépend du bon fonctionnement des vérins et du système d'alimentation de ceux-ci. En outre, le fonctionnement de vérins dans une ambiance, qui peut être agressive ou pour le moins à température élevée, est peu fiable.

On a également proposé dans le brevet américain US-A-2 629 312 d'assurer le maintien d'objets à l'intérieur d'un tambour tournant à l'aide de mécanismes de déplacement d'un plateau de pression, ce mécanisme étant actionné manuellement à l'aide d'une manivelle. Cette solution n'est guère satisfaisante dans la mesure où le milieu dans lequel il faut intervenir est hostile et dans la mesure où ces interventions manuelles ne permettent pas la mise en place d'une chaîne de traitement automatique des objets.

Pour remédier aux inconvénients mentionnés ci-dessus, un objet de l'invention est de fournir un dispositif de maintien d'objets dans un tambour tournant, notamment de stérilisateur ou appareils analogues, qui permette d'une part d'assurer un pressage efficace du produit ou des paniers contenant les produits de façon automatique et qui de plus assure ce serrage de façon fiable durant toute l'opération de traitement thermique correspondant, par exemple, à la stérilisation des produits.

Pour atteindre ce but, l'invention propose un dispositif de maintien d'objets à l'intérieur d'un tambour tournant, lesdits objets présentant une première face reposant sur un support solidaire dudit tambour et une deuxième face, ladite deuxième face étant sensiblement plane, ledit dispositif comprenant :

- un plateau de pression (22) présentant une face d'appui sensiblement plane ; et
- des moyens internes au tambour (10), pour déplacer ledit plateau entre une position de repos et une position de maintien dans laquelle ladite face d'appui du plateau est plaquée contre la deuxième face desdits objets, ledit dispositif se caractérisant en ce que les moyens de déplacement comprennent :
- une tige (30, 30') montée à rotation autour de son axe longitudinal (Y, Y') parallèle à l'axe de rotation (X, X') dudit tambour, et comportant n portions filetées (32 à 36, 130 à 136), au moins une portion ayant un sens de filetage opposé à celui de la ou des autres portions filetées ;
- n douilles taraudées (38 à 42, 138 à 144), chaque douille coopérant avec une des n portions filetées ;
- n systèmes à biellettes (48, 50, 146 à 150), chaque système comprenant deux biellettes et coopérant avec une douille et chaque système présentant une extrémité articulée sur ledit plateau (22);
- un moteur à fluide (60) couplé à la tige (30) pour provoquer la rotation de ladite tige selon deux sens de rotation lorsque le moteur est alimenté ; et
- des moyens d'alimentation (70, 72, 84, 86, 96, 100) du moteur en fluide sous pression, lesdits moyens d'alimentation traversant la paroi (14) dudit tambour sensiblement selon son axe de rotation (X, X'), par quoi dans un premier sens de rotation, les systèmes à biellettes amènent ledit plateau en position de repos et, dans un deuxième sens de rotation, lesdits systèmes à biellettes amènent ledit plateau en position de maintien.

On comprend qu'ainsi le pressage et le maintien en place des objets est obtenu par le déplacement du plateau, lui-même obtenu de façon automatique en commandant le moteur à fluide. En outre, on comprend que le maintien est assuré par le système à vis-écrou (douilles) et biellettes qui est sensiblement autobloquant en position de maintien, ce qui fait que, même en cas de défaillance du moteur à fluide, les objets demeurent efficacement maintenus en place.

Selon un premier mode de mise en oeuvre, chaque système à biellettes comprend une paire d'ensembles formant biellettes, chaque ensemble d'une même paire ayant une première extrémité articulée sur une desdites douilles et une deuxième extrémité articulée respectivement sur ledit plateau et sur un élément fixe solidaire dudit tambour.

Selon un deuxième mode de mise en oeuvre le dispositif se caractérise en ce qu'il comprend un nombre 2p (2p = n) de systèmes à biellettes, regroupés par deux, chaque système à biellettes comprenant une paire d'ensembles formant biellettes, chaque ensemble d'une même paire ayant une première extrémité articulée sur une desdites douilles et une deuxième extrémité articulée respectivement sur les deuxièmes extrémités des ensembles formant biellettes de la paire appartenant au même groupe, et une desdites deuxièmes extrémités étant en outre articulée sur ledit plateau.

D'autres caractéristiques et avantages de l'invention apparaîtront plus clairement à la lecture de la description qui suit de plusieurs modes de mise en oeuvre de l'invention donnés à titre d'exemple non limitatifs. La description se réfère aux figures annexées, sur lesquelles :

- la figure 1 est une vue en coupe verticale du tambour tournant montrant le mécanisme de maintien dans sa position de repos ;
- la figure 2 est une vue partielle du tambour similaire à celle de la figure 1 mais montrant le dispositif mécanique de maintien dans sa position de travail ;
- la figure 3 est une vue de droite du tambour de la figure 1 monté dans l'enceinte d'un autoclave de stérilisation ;
- la figure 4 est une vue partielle en coupe selon la ligne IV-IV de la figure 2 ;
- la figure 5 est une vue de détail de la partie F de la figure 1, vue de détail qui montre le passage des conduites d'alimentation du moteur à fluide vers la paroi du tambour ; et
- la figure 6 est une vue simplifiée d'un deuxième mode de réalisation des moyens de déplacement du plateau.

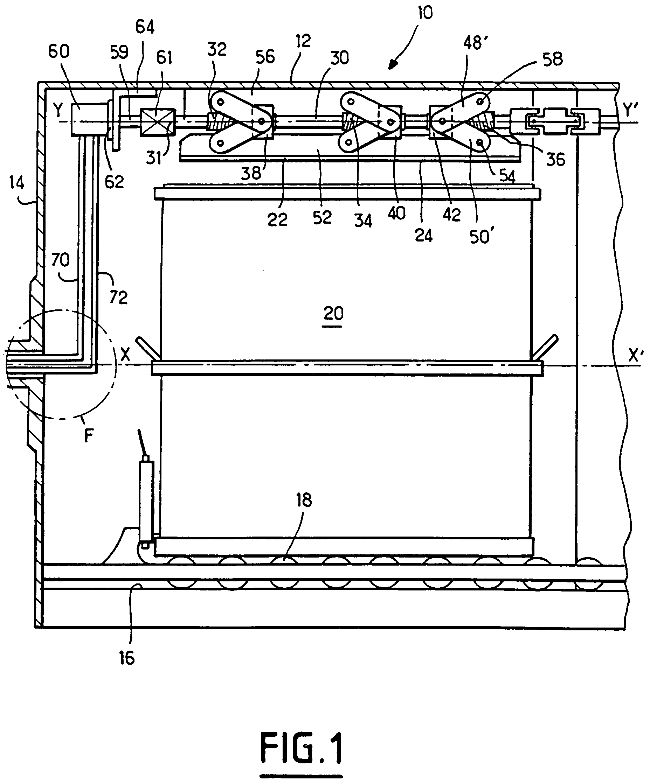

En se référant tout d'abord aux figures 1 à 4, on va décrire l'ensemble du tambour muni de son dispositif de maintien d'objets selon un premier mode de mise en oeuvre. Sur la figure 1, on a représenté le tambour 10 constitué par une paroi latérale 12 cylindrique dont l'axe XX' est en même temps l'axe de rotation du tambour 10, et par une paroi d'extrémité 14 circulaire dont la périphérie est soudée à la paroi latérale 12. A sa partie inférieure, le tambour est muni d'un plancher 16 sur lequel sont montés des rouleaux 18 pour faciliter l'introduction des paniers 20 dans lesquels sont placés les objets à stériliser.

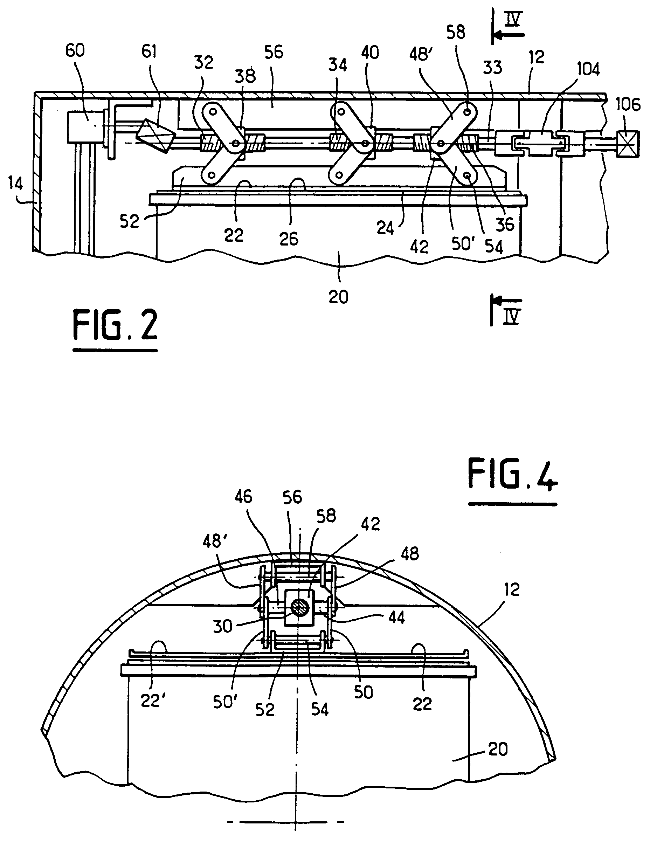

Le dispositif de maintien comprend un plateau mobile 22 qui peut être déplacé à l'aide d'un mécanisme entre une position de maintien telle que représentée sur la figure 2, dans laquelle le plateau 22 ou plus précisément sa face inférieure 24 est appliquée avec pression sur la face supérieure 26 du panier 20 et une position de repos représentée sur la figure 1 dans laquelle le plateau est écarté du sommet 26 du panier afin d'en permettre l'introduction ou l'extraction à l'intérieur ou hors du tambour.

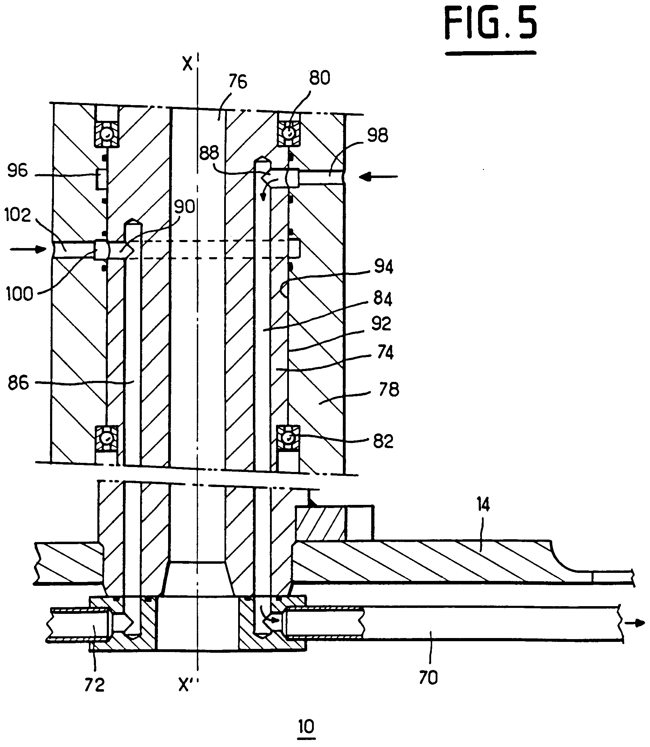

Le dispositif de déplacement est constitué par une tige 30 qui peut être mise en rotation autour de son axe longitudinal YY', l'axe YY' étant parallèle à l'axe de rotation XX' du tambour 10. La tige 30 comporte plusieurs portions filetées. Dans le cas de la figure 1, la tige comporte trois portions filetées référencées respectivement 32, 34 et 36. Sur chaque portion filetée est montée une douille taraudée, les douilles taraudées étant respectivement référencées 38, 40 et 42. De chaque côté des douilles 38 à 42 sont montés des demi-axes référencés 44 et 46 pour la douille 42 représentée sur la figure 4. Ces deux demi-axes 44 et 46 sont alignés. Sur chaque demi-axe 44, 46 est montée à pivotement une première extrémité de deux paires de biellettes respectivement référencées 48, 48' et 50, 50' pour la douille 42. Comme le montre les figures, une cornière en U 52 est fixée sur la face supérieure 22' du plateau 22 et disposée selon sa direction longitudinale. Des axes tels que 54 sont engagés dans des orifices ménagés dans les flancs de la cornière 52. Il y a autant d'axes que de douilles 38 à 42. De la même manière, une cornière en U 56 est solidaire de la partie sommitale de la paroi latérale 12 du tambour. Des axes tels que 58 sont engagés dans des orifices ménagés dans les flancs de la cornière 56, le nombre d'axes 58 étant également identique au nombre de douilles 38 à 42. Les deuxièmes extrémités des biellettes supérieures 48 et 50 sont engagées aux extrémités des axes supérieurs 58 et les deuxièmes extrémités des biellettes inférieures 48' et 50' sont engagées aux extrémités des axes tels que 54. On voit qu'on reconstitute ainsi trois systèmes de biellettes qui sont, d'une part, solidaires des douilles 38 à 42 et, d'autre part, respectivement solidaires de la cornière 56 liée au tambour et de la cornière 52 liée au plateau 22. Les filetages et taraudages associés des parties filetées 32 et 34 sont dans un premier sens, le filetage de la partie filetée 36 étant bien sûr dans un deuxième sens.

On comprend ainsi qu'en mettant en rotation autour de son axe YY' la tige 30, on provoquera l'écartement des paires de biellettes et donc la descente du plateau 22 vers sa position de maintien et qu'en faisant tourner la tige dans le sens opposé, on provoque le rapprochement des biellettes et le relèvement du plateau 22.

L'extrémité 31 de la tige 30 est accouplée à l'arbre 59 d'un moteur pneumatique 60 d'un type en soi connu, par l'intermédiaire d'un système 61 du type à joints de cardan. Le moteur 60 est muni d'une bride 62 qui est fixée sur une équerre 64 solidaire de la partie sommitale de la paroi latérale 12 du tambour.

On comprend que, grâce à la présence du système 61 à joint de cardan, le moteur 60 transmet le couple à la tige 30 dans toutes les positions qu'occupe celle-ci lors de sa rotation sous l'effet des biellettes 48, 48'.

Au lieu d'utiliser un moteur pneumatique, il serait possible de mettre en oeuvre un moteur hydraulique à condition de choisir un liquide de commande qui soit adapté aux conditions de température qui règnent à l'intérieur du tambour.

Le moteur 60 est alimenté par deux conduites 70 et 72 pour permettre les deux sens de rotation du moteur. Les deux conduites 70 et 72 sont alimentées en air comprimé par deux joints tournants qui sont mieux visibles sur la figure 5, qui va être décrite ci-après.

La paroi d'extrémité 14 du tambour 10 est solidaire d'un arbre 74 de mise en rotation disposé selon l'axe XX' du tambour. L'arbre 74 comporte un canal axial 76 pour l'introduction éventuelle de sondes dans le tambour. L'arbre 74 est monté à rotation dans un palier fixe 78 par l'intermédiaire de roulements 80 et 82. L'arbre 74 est en outre percé de deux conduites borgnes 84 et 86. L'extrémité borgne de chaque conduite est reliée à des canalisations radiales respectivement référencées 88 et 90. Les canalisations 88 et 90 débouchent dans la paroi externe 92 de l'arbre 74. En regard de la canalisation 88, la paroi interne 94 du palier 78 est munie d'une gorge circulaire 96 reliée elle-même à une canalisation radiale d'alimentation 98 ménagée dans le palier fixe 78. Symétriquement, en regard de la canalisation 90, la paroi interne 94 du palier 78 est munie d'une gorge circulaire 100 reliée à une deuxième canalisation d'alimentation 102. Des joints toriques d'étanchéité 104, 106, 108, 110 sont montés de part et d'autre des gorges annulaires 96 et 100. Comme le montre la figure 5, l'extrémité ouverte des canalisations 84 et 86 est reliée aux extrémités des tubulures 70 et 72. Ainsi, par ce double système à joints tournants, le moteur 60 peut être alimenté en permanence soit par l'air comprimé appliqué à la canalisation fixe 102, soit par l'air comprimé appliqué à la canalisation fixe 98.

Si l'on revient aux figures 1 et 2, on voit que la deuxième extrémité 33 de la tige 30 peut être reliée, via un système à joint de cardan 104, à un organe manuel de mise en rotation 106 constitué, par exemple, par une manivelle. Ainsi, même en cas de défaillance du moteur 60, il est possible de commander manuellement la rotation de la tige et donc les mouvements du plateau 22.

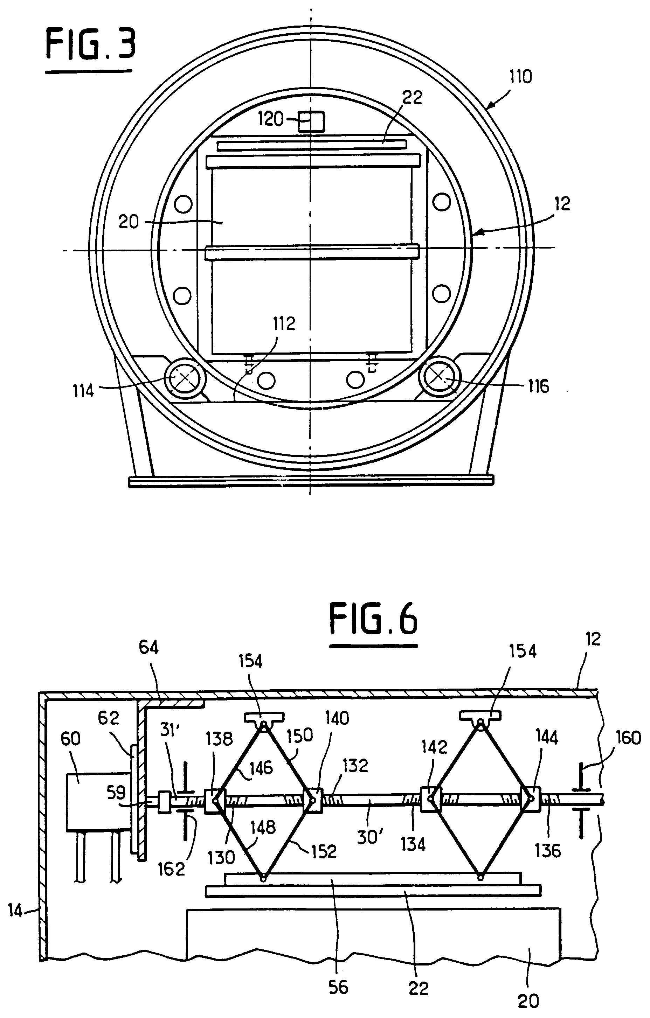

La figure 3 montre le tambour 10 monté dans l'enceinte autoclave d'un stérilisateur. Cette figure montre l'enveloppe externe 110 du stérilisation avec son "plancher" 112 sur lequel sont montés deux rouleaux supports 114 et 116 montés fous pour guider la paroi latérale 12 du tambour 10 lors de sa rotation. Sur la figure 3 on a également fait apparaître les paniers 20 contenant les produits à stériliser, le plateau presseur 22 et, sous une forme symbolique, le dispositif 120 de déplacement du plateau 22.

La figure 6 montre sous une forme simplifiée un deuxième mode de réalisation des moyens de déplacement du plateau 22. La tige 30' comporte quatre portions filetées 130, 132, 134 et 136 à pas alternativement inversés. Sur les portions filetées sont montées quatre douilles 138, 140, 142 et 144. Sur chaque douille sont articulées les premiers extrémités de deux biellettes, 146 et 148 pour la douille 138 et 150 et 152 pour la douille 140. Les deuxièmes extrémités des biellettes 146 et 150 sont articulées entre elles et sur un "sabot" 154. Symétriquement les deuxièmes extrémités des biellettes 148 et 152 sont articulées entre elles et sur la cornière 56 solidaire du plateau 22. Les biellettes 156 à 162 associées aux douilles 142 et 144 sont montées de la même manière. On constitue ainsi deux parallèlogrammes déformables dont la configuration est commandée par la position relative des groupes de douilles 130, 132 et 134, 136.

L'extrémité 31' de la tige 30' est directement solidaire de l'extrémité de l'arbre 59 du moteur pneumatique 60. Celui-ci est fixé sur l'équerre 64 par l'intermédiaire de la bride 62. On comprend qu'en commandant le moteur 60 dans un premier sens, les sommets des parallélogrammes portant les sabots 154 et le plateau 22 s'écartent mutuellement jusqu'à ce que les sabots 154 viennent au contact de la partie sommitale de la paroi latérale 12 du tambour, et que simultanément le plateau 22 vienne se plaquer contre la face supérieure des paniers 20. Les paniers sont alors parfaitement maintenus. En commandant la rotation du moteur 60 dans l'autre sens, on provoque le rapprochement des sabots 154 et du plateau 22 vers la tige 30', ce qui libère les paniers 20.

On comprend que ce deuxième mode de réalisation présente les mêmes avantages que le premier mode de réalisation. Un avantage supplémentaire réside dans le fait que la tige 30' est immobile en translation, ce qui permet de monter ses extrémités dans deux paliers 160 et 162 solidaires du tambour 10. En revanche il est nécessaire de réaliser un positionnement très précis de la tige 30' pour que les sabots 154 viennent en butée contre la paroi 12 en même temps que le plateau 22 vient au contact de la face supérieure des paniers 20. Celà implique que ce deuxième mode de réalisation est très bien adapté au cas où les objets qui sont chargés dans le tambour rotatif ont toujours la même hauteur. Au contraire, dans le cas du premier mode de réalisation, le dispositif de maintien s'adapte à différentes hauteurs d'objets.

Dans la description précédente, on a seulement considéré le cas où le dispositif de maintien comprend un seul plateau de pression. Il va de soi qu'on ne sortirait pas de l'invention si la tige 30 ou 30' servait à commander, par l'intermédiaire de systèmes à biellettes, plusieurs plateaux de pression séparés.

## Revendications

1. Dispositif de maintien d'objets à l'intérieur d'un tambour tournant, lesdits objets, présentant une première face reposant sur un support solidaire dudit tambour et une deuxième face, ladite deuxième face étant sensiblement plane, ledit dispositif comprenant :

- un plateau de pression (22) présentant une face d'appui sensiblement plane ; et
- des moyens internes au tambour (10), pour déplacer ledit plateau entre une position de repos et une position de maintien dans laquelle ladite face d'appui du plateau est plaquée contre la deuxième face desdits objets, ledit dispositif se caractérisant en ce que les moyens de déplacement comprennent :
- une tige (30, 30') montée à rotation autour de son axe longitudinal (Y, Y') parallèle à l'axe de rotation (X, X') dudit tambour, et comportant n portions filetées (32 à 36, 130 à 136), au moins une portion ayant un sens de filetage opposé à celui de la ou des autres portions filetées ;
- n douilles taraudées (38 à 42, 138 à 144), chaque douille coopérant avec une des n portions filetées ;
- n systèmes à biellettes (48, 50, 146 à 150), chaque système comprenant deux biellettes et coopérant avec une douille et chaque système présentant une extrémité articulée sur ledit plateau (22);
- un moteur à fluide (60) couplé à la tige (30) pour provoquer la rotation de ladite tige selon deux sens de rotation lorsque le moteur est alimenté ; et
- des moyens d'alimentation (70, 72, 84, 86, 96, 100) du moteur en fluide sous pression, lesdits moyens d'alimentation traversant la paroi (14) dudit tambour sensiblement selon son axe de rotation (X, X'), par quoi dans un premier sens de rotation, les systèmes à biellettes amènent ledit plateau en position de repos et, dans un deuxième sens de rotation, lesdits systèmes à biellettes amènent ledit plateau en position de maintien.

2. Dispositif de maintien selon la revendication 1, caractérisé en ce que chaque système à biellettes comprend une paire d'ensembles formant biellettes (48, 48', 50, 50'), chaque ensemble d'une même paire ayant une première extrémité articulée sur une desdites douilles (38 à 42) et une deuxième extrémité articulée respectivement sur ledit plateau (22) et sur un élément fixe (56) solidaire dudit tambour (10).

3. Dispositif de maintien selon la revendication 1, caractérisé en ce qu'il comprend un nombre 2p (2p = n) de systèmes à biellettes, regroupés par deux, chaque système à biellettes comprenant une paire d'ensembles formant biellettes (146, 148 ; 150, 152), chaque ensemble d'une même paire ayant une première extrémité articulée sur une desdites douilles (138, 140) et une deuxième extrémité articulée respectivement sur les deuxièmes extrémités des ensembles formant biellettes (142, 144) de la paire appartenant au même groupe, et une desdites deuxièmes extrémités étant en outre articulée sur ledit plateau (22).

4. Dispositif de maintien selon la revendication 2, caractérisé en ce que ladite tige (30) est libre en translation selon la direction de déplacement dudit plateau, en ce que ledit moteur (60) à fluide est solidaire dudit tambour (10) et en ce que l'arbre dudit moteur (59) est relié à une extrémité (31) de ladite tige par un système mécanique (61) apte à transmettre un mouvement de rotation tant en autorisant ledit mouvement relatif de translation entre ledit arbre et ladite tige.

5. Dispositif de maintien selon la revendication 3, caractérisé en ce que ladite tige (30') est immobile en translation, en ce que ledit moteur (60) est solidaire dudit tambour et en ce que l'arbre dudit moteur (59) est directement relié à une extrémité (31') de ladite tige.

6. Dispositif de maintien selon l'une quelconque des revendications 1 à 5, caractérisé en ce que l'extrémité (33) de ladite tige (30) non reliée au moteur, peut être reliée à un organe (106) de mise en rotation manuelle de ladite tige.

7. Dispositif de maintien selon l'une quelconque des revendications 1 à 6, caractérisé en ce que lesdits moyens d'alimentation du moteur comprennent au moins deux conduites (70, 72) internes audit tambour reliées à deux canalisations (84, 86) ménagées dans l'arbre (74) d'entraînement en rotation du tambour, chacune des canalisations étant alimentée par un système à joint tournant (90, 100 ; 88, 96).

8. Dispositif de maintien selon l'une quelconque des revendications 1 à 7, caractérisé en ce que ledit moteur (60) est un moteur pneumatique.

**Claims**

1. Device for holding objects inside a rotating drum, said objects having a first face resting on a support fast with said drum and a second face, said second face being substantially plane, said device comprising:

   - a pressure plate (22) having a substantially plane bearing face; and
   - means inside the drum (10) for displacing said plate between a rest position and a holding position in which said bearing face of the plate is applied against the second face of said objects, said device being characterized in that the displacement means comprise:
   - a rod (30, 30') mounted to rotate about its longitudinal axis (Y, Y') parallel to the axis of rotation (X, X') of said drum, and comprising n threaded portions (32 to 36, 130 to 136), at least one portion having a direction of threading opposite that of the other threaded portion or portions;

- n tapped sleeves (38 to 42, 138 to 144), each sleeve cooperating with one of the n threaded portions;

- n link arm systems (48, 50, 148 to 150), each system comprising two link arms and cooperating with a sleeve and each system having one end articulated on said plate (22);

- a fluid motor (60) coupled to the rod (30) to provoke rotation of said rod in two directions of rotation when the motor is supplied; and

- means (70, 72, 84, 86, 96, 100) for supplying the motor with fluid under pressure, said supply means traversing the wall (14) of said drum substantially along its axis of rotation (X, X'), whereby, in a first direction of rotation, the link arm systems bring said plate into rest position and, in a second direction of rotation, said link arm systems bring said plate into a holding position.

2. Holding device according to Claim 1, characterized in that each link arm system comprises a pair of assemblies forming link arms (48, 48', 50, 50'), each assembly of the same pair having a first end articulated on one of said sleeves (38 to 42) and a second end articulated respectively on said plate (22) and on a fixed element (58) fast with said drum (10).

3. Holding device according to Claim 1, characterized in that it comprises a number 2p (2p = n ) of link arm systems, grouped in pairs, each link arm system comprising a pair of assemblies forming link arms (146, 148; 150, 152), each assembly of the same pair having a first end articulated on one of said sleeves (138, 140) and a second end articulated respectively on the second ends of the assemblies forming link arms (142, 144) of the pair belonging to the same group, and one of said second ends being further articulated on said plate (22).

4. Holding device according to Claim 2, characterized in that said rod (30) is free in translation in the direction of displacement of said plate, in that said fluid motor (80) is fast with said drum (10) and in that the shaft of said motor (59) is connected to one end (31) of said rod by a mechanical system (61) adapted to transmit a movement of rotation while allowing said relative movement of translation between said shaft and said rod.

5. Holding device according to Claim 3, characterized in that said rod (30') is fixed in translation, in that said motor (60) is fast with said drum and in that the shaft of said motor (59) is directly connected to one end (31') of said rod.

6. Holding device according to any one of Claims 1 to 5, characterized in that the end (33) of said rod (30) not connected to the motor may be connected to a member (106) for manually rotating said rod.

7. Holding device according to any one of Claims 1 to 6, characterized in that said means for supplying the motor comprise at least two conduits (70, 72) inside said drum connected to two pipes (84, 86) arranged in the shaft (74) for driving the drum in rotation, each one of the pipes being supplied by a rotary joint system (90, 100; 88, 96).

8. Holding device according to any one of Claims 1 to 7, characterized in that said motor (80) is a pneumatic motor.

**Patentansprüche**

1. Vorrichtung zur Halterung von Gegenständen im Inneren einer Drehtrommel, welche Gegenstände eine auf einem mit der Trommel fest verbundenen Träger ruhende erste Fläche und eine zweite Fläche aufweisen, wobei die zweite Fläche im wesentlichen plan ist, welche Vorrichtung folgendes umfaßt:

- eine Druckplatte (22) mit einer im wesentlichen planen Auflagefläche; und
- Mittel innerhalb der Trommel (10) zum Verschieben der Platte zwischen einer Ruheposition und einer Halterungsposition, in welcher die Auflagefläche der Platte an der zweiten Fläche der Gegenstände anliegt, welche Vorrichtung dadurch gekennzeichnet ist, daß die Verschiebemittel umfassen:
- eine Stange (30, 30'), die drehbar um ihre zur Rotationsachse (X, X') der Trommel parallele Längsachse (Y, Y') angeordnet ist und n Gewindeabschnitte (32 bis 36, 130 bis 136) aufweist, wobei mindestens ein Abschnitt eine Gewinderichtung aufweist, die zu jener des bzw. der anderen Gewindeabschnitt(e) entgegengesetzt ist;
- n Gewindebuchsen (38 bis 42, 138 bis 144), wobei jede Buchse mit einem der n Gewindeabschnitte zusammenwirkt;
- n Schwingarmsysteme (48, 50, 146 bis 150), wobei jedes System zwei Schwingarme aufweist und mit einer Buchse zusammenwirkt und jedes System ein Ende besitzt, welches an der Platte (22) angelenkt ist;
- einen Fluidmotor (60), welcher an die Stange (30) gekuppelt ist, um die Rotation der Stange in zwei Rotationsrichtungen zu bewirken, wenn der Motor beaufschlagt ist; und
- Speiseeinrichtungen (70, 72, 84, 86, 96, 100) für den Druckmittelmotor, welche Speiseeinrichtungen im wesentlichen gemäß der Rotationsachse (X, X') der Trommel durch die Wand (14) derselben hindurchtreten, wobei in einer ersten Drehrichtung die Schwingarmsysteme die Platte in die Ruheposition bringen und in einer zweiten Drehrichtung die Schwingarmsysteme die Platte in die Halterungsposition bringen.

2. Halterungsvorrichtung nach Anspruch 1, dadurch gekennzeichnet, daß jedes Schwingarmsystem ein Paar von Schwingarme (48, 48', 50, 50') bildenden Einheiten aufweist, wobei jede Einheit ein- und desselben Paares ein erstes Ende an einer der Buchsen (38 bis 42) und ein zweites Ende an der Platte (22) bzw. an einem mit der Trommel (10) fest verbundenen fixen Element (56) angelenkt hat.

3. Halterungsvorrichtung nach Anspruch 1, dadurch gekennzeichnet, daß sie eine Anzahl 2p (2p = n) von Schwingarmsystemen aufweist, die paarweise gruppiert sind, wobei jedes Schwingarmsystem ein Paar von Schwingarme (146, 148; 150, 152) bildenden Einheiten aufweist und jede Einheit ein- und desselben Paares ein erstes Ende an einer der Buchsen (138, 140) und ein zweites Ende jeweils an den zweiten Enden der Schwingarme (142, 144) bildenden Einheiten des zur selben Gruppe gehörenden Paares angelenkt hat und eines der zweiten Enden außerdem an der Platte (22) angelenkt ist.

4. Halterungsvorrichtung nach Anspruch 2, dadurch gekennzeichnet, daß die Stange (30) frei beweglich in der Verschieberichtung der Platte ist, daß der Fluidmotor (60) mit der Trommel (10) fest verbunden ist und daß die Welle (59) des Motors durch ein mechanisches System (61), welches eine Rotationsbewegung übertragen kann, dabei aber die Relativbewegung zwischen der Welle und der Stange zuläßt, mit einem Ende (31) der Stange verbunden ist.

5. Halterungsvorrichtung nach Anspruch 3, dadurch gekennzeichnet, daß die Stange (30') in der Translationsbewegung immobilisiert ist, daß der Motor (60) mit der Trommel fest verbunden ist und daß die Welle (59) des Motors direkt mit einem Ende (31') der Stange verbunden ist.

6. Halterungsvorrichtung nach einem der Ansprüche 1 bis 5, dadurch gekennzeichnet, daß das Ende (33) der Stange (30), welches nicht mit dem Motor verbunden ist, an ein Organ (106) zum händischen Versetzen der Stange in Rotation verbindbar ist.

7. Halterungsvorrichtung nach einem der Ansprüche 1 bis 6, dadurch gekennzeichnet, daß die Speiseeinrichtungen des Motors mindestens zwei innerhalb der Trommel befindliche Leitungen (70, 72) aufweisen, die mit zwei Kanälen (84, 86) verbunden sind, welche in der Welle (74) zum Versetzen der Trommel in Rotation vorgesehen sind, wobei jeder der Kanäle durch ein System mit Drehverbindung (90, 100; 88, 96) gespeist ist.

8. Halterungsvorrichtung nach einem der Ansprüche 1 bis 7, dadurch gekennzeichnet, daß der Motor (60) ein pneumatischer Motor ist.

FIG.1

FIG. 2

FIG. 4

## FIG.3

## FIG.6

# FIG.5

10